# EUROPEAN PATENT SPECIFICATION

(11) **EP 4 142 762 B1**
(45) Date of publication and mention of the grant of the patent: **29.01.2025**
(21) Application number: 21728115.3
(22) Date of filing: 29.04.2021
(51) Int. Cl.: A61K 36/185, A61K 36/31, A61K 36/52, A61K 36/54, A61K 36/73, A61K 36/889, A61P 39/00

(54) **SUPERFOOD FOR PROLONGING THE LONGEVITY OF AN INDIVIDUAL**
SUPERNAHRUNGSMITTEL ZUR VERLÄNGERUNG DER LEBENSDAUER EINER PERSON
SUPERALIMENT POUR PROLONGER LA LONGÉVITÉ D'UN INDIVIDU

(30) Priority: 30.04.2020 IT 202000009610
(43) Date of publication of application: 08.03.2023
(73) Proprietor: Giuliani S.p.A., 20129 Milano (IT)
(72) Inventor: GIULIANI, Giammaria, 6926 Montagnola (CH); RINALDI, Fabio, 20129 Milano (IT); PINTO, Daniela, 20151 Milano (IT); MARZANI, Barbara, 27020 Carbonara Al Ticino (IT); GOBBETTI, Marco, 06134 Perugia (IT); DI CAGNO, Raffaella, 70126 Bari (IT); FIORINO, Giuseppina Maria, 87030 Cosenza (IT); TLAIS, Ali Zein Alabiden, 39100 Bolzano (IT)
(74) Representative: Cattaneo, Elisabetta
(86) International application number: PCT/IB2021/053567
(87) International publication number: WO 2021/220211

(56) References cited:
- US-A1- 2020 030 401
- KAJISA I: "Preparing smoothie used for rejuvenating body and providing anti-aging effect, by mixing large sized-boiled broccoli, large sized-avocado, unpeeled apple, and cow's milk in mixer, and adding nicotinamide mononucleotide and quercetin", WPI / 2017 CLARIVATE ANALYTICS,, vol. 2018, no. 41, 7 June 2018 (2018-06-07), XP002801390
- DATABASE WPI Week 201841, Derwent World Patents Index; AN 2018-46113P, XP002801390
- ESSA M M ET AL: "Neuroprotective effect of natural products against Alzheimer's disease", NEUROCHEMICAL RESEARCH 2012 SPRINGER NEW YORK USA, vol. 37, no. 9, September 2012 (2012-09-01), pages 1829 - 1842, XP002801391, ISSN: 0364-3190

## Description

### Field of the Invention

The present invention relates to a nutritional composition for promoting the longevity of an individual and/or maintaining the well-being of an individual.

The present invention originates in the nutraceutical and pharmaceutical sectors. In particular, the present invention relates to a nutritional composition based on mixtures of selected vegetables or vegetal extracts which maintain in physiological conditions the cellular processes that are the basis of longevity of an individual.

### State of the Art

The most current research in the nutritional field is aimed at identifying strategies for modulating the diet in order to reduce the risk of developing chronic diseases and increase life expectancy and longevity of individuals.

Numerous research projects on protective factors for human health and new nutritional approaches aimed at preventing conditions that can favour the development of diseases have therefore been launched by researchers, nutritionists, and physicians. A large portion of these nutritional approaches are based on compliance with dietary regimes, whose observance reduces the risk of developing chronic diseases that may compromise an individual's health and reduce life expectancy.

A large portion of the international scientific community is inclined to believe that some of the main causes of death, such as cardiovascular or respiratory diseases, diabetes, and the development of certain cancers, are the result of a process that originates in childhood and is influenced by the dietary regime followed.

It was also observed that, by implementing behavioural changes oriented towards regular physical exercise, suitable stress management and a balanced eating style, that can also benefit from the use of food supplements, it is possible to delay or in some cases avoid the onset of numerous diseases whose appearance reduces life expectancy of an individual.

Currently, sophisticated technologies are also applied with the aim of intervening on the biological phenomenon of an organism's aging with the aim of improving life quality and expectancy. These sophisticated technologies also make use of genetic studies aimed at early determination of the main individual risk factors, and the use of biotechnologies aimed at providing means to intervene on certain problems, thanks to the use of gene therapies. However, these sophisticated technologies have the drawback of entailing high costs and being therefore aimed only at a very limited segment of the population.

There is currently a need to have new methods leading to the creation of products that are easily accessible to a large portion of the population, whose use would make it possible to positively intervene on longevity of individuals.

One of the objects of the invention is therefore to provide a nutritional composition based on food substances of vegetal origin or vegetal extracts whose intake positively affects the longevity of a human individual.

Another object of the invention is to provide a nutritional composition based on a mixture of selected edible vegetables or vegetal extracts whose intake or supplementation in a dietary regime helps to slow down the human body aging.

A further object is to provide a method for prolonging the maintenance of physiological conditions in a human being organism.

### Summary of the Invention

Within the technical field of the invention, the Applicant has found that by combining a basic mixture of nutritional components of vegetal origin, or extracts thereof, with two selected further vegetal components, or extracts thereof, nutritional compositions are obtained that activate certain biological mechanisms, in particular mechanisms for regulating the activity of sirtuins, SIRT1 and SIRT3, and a protein kinase named AMPK (AMP-activated protein kinase) that promote the longevity of an individual. Sirtuins are a highly conserved class of aging regulators. AMPK acts as an energy sensor, since it perceives the increase in AMP concentration and becomes activated, thus increasing the intracellular levels of NAD+, and consequently stimulating the activity of SIRT1.

In particular, the inventors have experimentally found that by combining, in specific ratios, a mixture of vegetables belonging to the Moringa, Pomegranate and Walnut species, or extracts thereof, with a vegetable selected from Avocado and/or Broccoli species, or an extract thereof, nutritional compositions are obtained that increase the longevity of an individual and/or contribute to maintaining the health state or physiological conditions of an individual.

In the context of the present invention, the terms "vegetable" or "vegetal component(s)" are to be understood as synonyms and referred to the five vegetal species or plants described herein. Of these five vegetal species, three are essential basic components that can be added to one or both of two additional components.

The term "basic mixture of nutritional components of vegetal origin" means the mixture of the three vegetal nutritional components of Moringa, Pomegranate and Walnut.

Suitable nutritional parts of the plants that make up the composition of the invention include leaves, seeds, fruits, roots, or the trunk/stem. The fruits of the selected five vegetal species are preferably used.

For example, when the vegetal species is Pomegranate, Walnut, or Avocado the fruits are used, when it is Moringa the leaves are used to prepare the composition. According to some embodiments of the invention, the vegetable or vegetal components contained in the composition described herein can be in the form of vegetal extracts.

In view of the objects set out above, the present invention concerns a nutritional composition comprising the three vegetal components of Pomegranate, Walnut and Moringa in combination with one further vegetal nutritional component selected from Avocado and Broccoli, wherein, advantageously, the vegetal components of Pomegranate, Walnut, Moringa are in a ratio or proportion of 2.7-3.3 : 2.7-3.3 : 1.7-2.3, preferably 3:3:2 and the additional nutritional component is in a ratio or proportion of 1.7-2.3, preferably :2.

The combination of the selected vegetal components results in a synergistic activation of sirtuins, in particular *SIRT-1, SIRT-3,* and *AMPK-1* that promote the longevity of an individual.

According to a general aspect, the invention relates to a nutritional composition as defined in the appended claim 1 and uses thereof as defined in claims 10,12.

According to certain preferred embodiments, the nutritional compositions are the following two, each containing 4 main components of vegetal origin:
Composition 1: comprising a mixture of Pomegranate, Walnut and Moringa combined with Broccoli, wherein the four components are preferably in a ratio of 3:3:2:2.
Composition 2: comprising a mixture of Pomegranate, Walnut and Moringa combined with Avocado, wherein the four components are preferably in a ratio of 3:3:2:2.

Advantageously, the compositions of the invention contain the vegetal components in shredded and/or dehydrated form, if desired.

In some embodiments of the invention, said vegetal components are treated with extraction techniques that can be applied to both fresh and dehydrated vegetal matter that can be advantageously ground or pulverized in order to facilitate extraction.

The composition of the invention may contain the vegetable as such, for example a part thereof, or as an extract.

The vegetable may be used either in the whole form or a portion thereof containing biologically active components. Alternatively, the composition contains extracts of the vegetables / vegetal species described herein.

The extraction can be carried out on a vegetal portion of the plant of interest, vegetal tissues thereof, or one of its parts selected from root, stem, leaf, flower, inflorescence, fruit, preferably fruit.

Preferably, an initial step of the preparation of suitable vegetal extracts provides that the plant/vegetable of interest, or parts thereof, are cleaned, dried and optionally cut into pieces or shredded, ground or pulverized. In a later step, this material is extracted using conventional methods.

The extraction can be carried out using a physiologically acceptable solvent in which the biologically active components are soluble and do not undergo any alterations depriving them of activity.

In some embodiments the solvent is hydrophilic. The solvent can also be an organic solvent. If it is desired to concentrate the extract for the benefit of one rather than another biologically active substance, solvents with different polarity can be useful. A suitable solvent to obtain the vegetal extract of one of the vegetables/plants described herein is a physiologically acceptable liquid in which the biologically active components are soluble, and they do not undergo any alterations depriving them of activity.

The extraction can be carried out with a suitable solvent such as water, or an alcohol such as ethanol, or a hydroalcoholic mixture such as water-ethanol.

By way of example, the hydroalcoholic solvent may contain from 10% to 70% by volume of alcohol, preferably ethanol, preferably from 20% to 60% by volume, still preferably from 30% to 50% by volume, for example 40% by volume.

In certain embodiments, the extraction of one or more biologically active components from the vegetable of interest occurs by maceration of the fruits of the plant, in water, ethanol or a hydroalcoholic mixture, for example containing from 10% to 70% by volume of alcohols, preferably ethanol, preferably from 20% to 60% by volume, still preferably from 30% to 50% by volume, for example 40% by volume. The extraction can be a cold or hot extraction, for example at 40-70 degrees centigrade.

The extracted liquid can be filtered, when necessary. At a later stage, the liquid extract is dried to obtain the biologically active component in the form of a dry powder. Drying can be achieved with conventional techniques, such as air, heating or spray-drying.

Suitable methods for obtaining the vegetal extract from one of the vegetables/vegetal species of interest include extraction techniques by digestion, infusion, pressing, maceration, decoction, percolation, distillation, counter-current extraction, extraction with supercritical gas or ultrasound.

Typically, in the extraction step the plant part of interest, for example the fruits, are contacted with the solvent for a variable time to obtain the extraction of an effective amount of biologically active component. In certain embodiments, the extraction time may vary between 1 and 48 hours.

According to certain embodiments, the preparation of a suitable extract comprises the following steps:
- shredding a portion of the vegetable, for example fruit or leaves,
- addition of a hydroalcoholic extraction solvent, such as ethanol, for example to obtain a vegetable/hydroalcoholic solvent ratio by weight ranging from about 1:10 to about 1:50,
- maceration,
- extraction,
- filtration,
- optional concentration of the filtrate, for example at reduced pressure, by evaporation of the solvent.

In certain embodiments, the extraction step can be repeated two or three times, until the material to be extracted is exhausted.

The extracted material can then be dried to give a dry extract.

For the purposes of the present invention, the vegetal extracts obtained by means of these techniques can be added to carriers or suitable adjuvants, such as maltodextrins, glucose or iso-glucose syrups, propylene glycol, glycerin or oily carriers such as, for example, medium-chain triglycerides or vegetable oils.

Extracts can be used to obtain more concentrated preparations of the substances of interest, selected for their physiological effect, to be used in pre-dosed forms or in foods.

According to one aspect, a kit comprising a basic mixture of vegetal components comprising Pomegranate, Walnut and Moringa, or extracts thereof, and one further vegetal component selected from Avocado, Broccoli, or extracts thereof, is provided to promote the longevity of an individual.

In one aspect, the invention provides a method for increasing the longevity of an individual comprising mixing vegetal components/fruit of Pomegranate, Walnut and Moringa with at least one further vegetal component selected from Avocado, Broccoli, and, advantageously in proportions of 2.7-3.3 : 2.7-3.3 : 1.7-2.3 : 1.7-2.3, preferably 3:3:2:2.

The vegetal components used within the scope of the invention are parts of selected vegetal species or selected plant fruits.

According to some aspects, the composition of the invention is indicated in preventing and/or treating premature aging of an individual and/or promoting the longevity of an individual.

The fruit and vegetable matrices used in the present invention have characteristics that, when combined, determine an enhancement of the activities of the single vegetal components.

In the present context, the term fruit or vegetable "matrices" means the vegetable or fruit portion used to prepare the composition described herein.

The authors of the present invention have identified specific vegetal components described herein, whose combination determines health properties superior to those obtainable with single vegetal components/ingredients.

Further advantages of the present invention are: (i) the possibility of using various, relatively low cost, fruit and vegetable natural matrices that compensate for the consumption of nutrient-rich sources to adhere to the main recommendation of a healthy Mediterranean diet; (ii) the unusual content, such as unsaturated fatty acids, minerals and fibers, compared to most of the products available on the market; (iii) the possibility of extending the consumption of these preparations to populations all over the world, in particular to those people who have nutritional deficiencies due to daily eating habits; and (iv) the consumption of fruit and vegetable preparations with unexpectedly high anti-aging activities.

The composition of the invention can be administered orally and in some forms for topical application. In the latter case, the composition of the invention described herein finds application in the cosmetic or aesthetic fields in preventing premature aging of the skin and/or in treating skin damage caused by the action of external agents, in particular prolonged exposure to UVs rays. In the production of topical products, the use of extracts is definitely advantageous compared to shredded and dehydrated fruit and vegetables.

### Brief Description of the Figures

Features and advantages of the present invention will become more fully apparent from the attached drawing tables wherein:

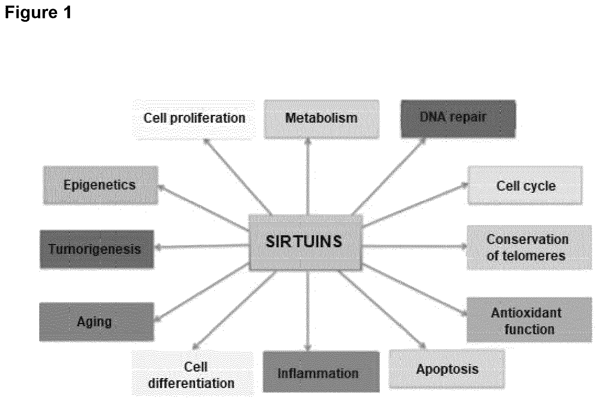

Figure 1 shows the role played by Sirtuins in multiple biological processes in the human body.

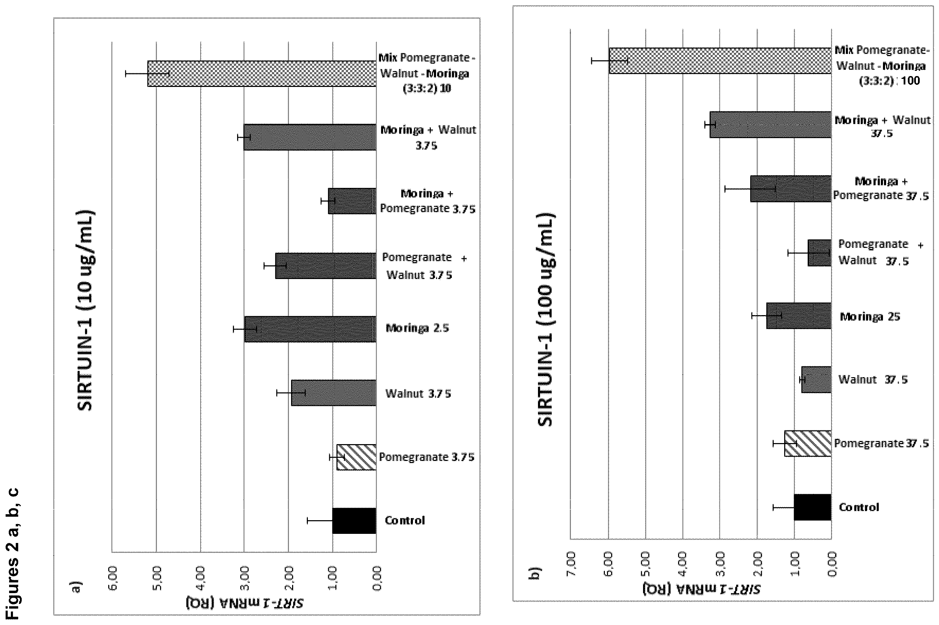

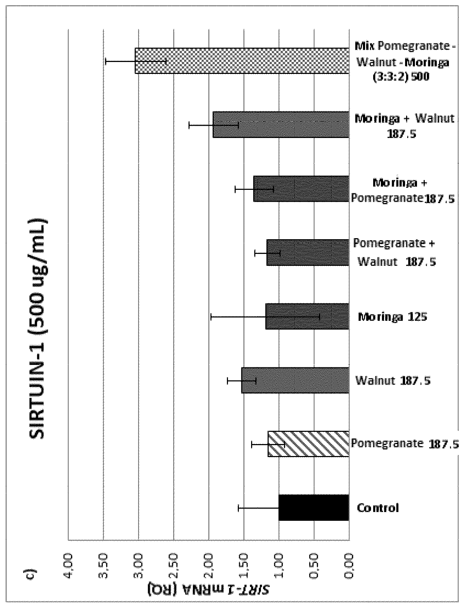

Figures 2a, b, c, show bar graphs related to SIRT-1 gene expression in NCTC2544 human keratinocytes as determined by RT-PCR. NCTC2544 cells were treated for 24 hours at 37°C, 5% CO₂, with: basal medium containing 2.5% FBS (vehicle); the vegetal components Pomegranate; Walnut; Moringa, singly and as mixture of two, and as mixture of three (basic mixture). The mixture of the three components is tested at 10 µg/mL (a), 100 µg/mL (b) and 500µg/mL (c), respectively, and the active vegetal components were also tested at a concentration reflecting that in the final combination. The values are the average of 2 duplicate experiments.

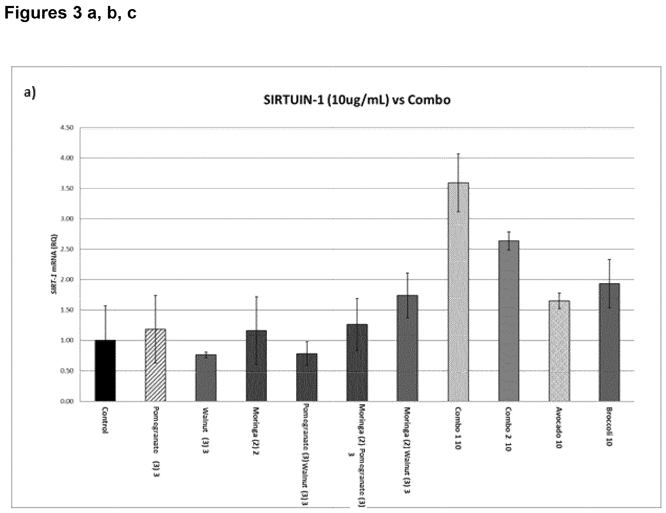

Figures 3a), b), c) show bar graphs illustrating SIRT-1 gene expression in NCTC2544 human keratinocytes as determined by RT-PCR. NCTC2544 cells were treated for 24 hours at 37°C, 5% CO₂, with: basal medium containing 2.5% FBS (vehicle); Pomegranate; Walnut; Moringa, singly and as mixture of two; Avocado; and Broccoli singly, and the combinations Combo 1; Combo 2 of the invention. The combination compositions of the invention having a ratio of 3:3:2:2 were tested at 10 µg/mL (a), 100 µg/mL (b) and 500µg/mL (c), respectively, and the active vegetal components were also tested at a concentration reflecting that in the final combination. The values are the average of 2 duplicate experiments.

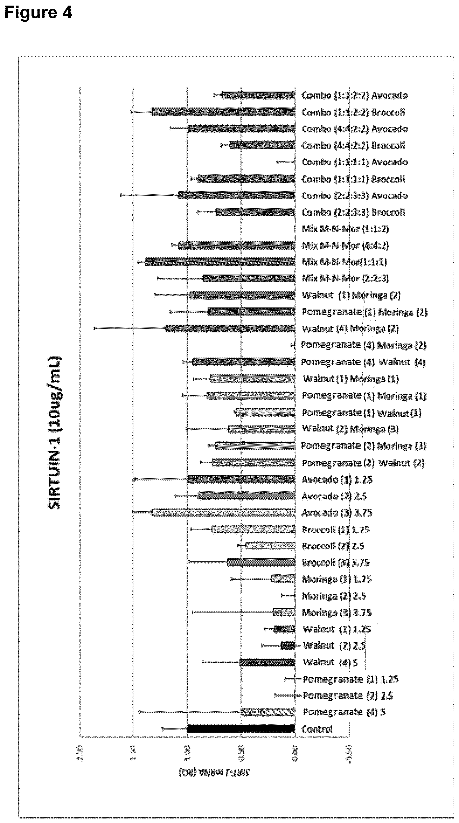

Figure 4 shows bar graphs representing *SIRT-1* gene expression in human NCTC2544 keratinocytes as determined by RT-PCR. NCTC2544 keratinocytes were treated for 24 hours at 37°C, 5% CO₂, with: basal medium containing 2.5% FBS (vehicle); the single vegetal components Pomegranate; Walnut; Moringa, two-component mixtures thereof, the basic mixture of the three vegetal components, and the compositions of the invention (Combo) comprising the basic mixture of the three vegetal components and one of Avocado or Broccoli in different proportions. The combinations (2:2:3:3; 1:1:1:1; 4:4:2:2; 1:1:2:2) were tested at 10µg/mL and the active vegetal components were also tested at a concentration reflecting that in the final combination. The values are the result of 2 duplicate experiments.

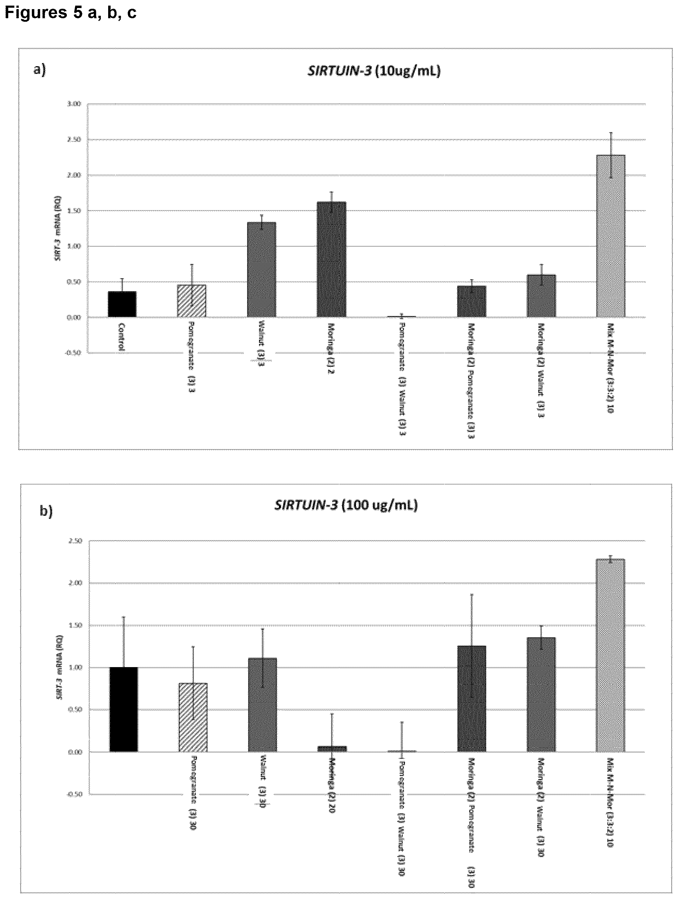

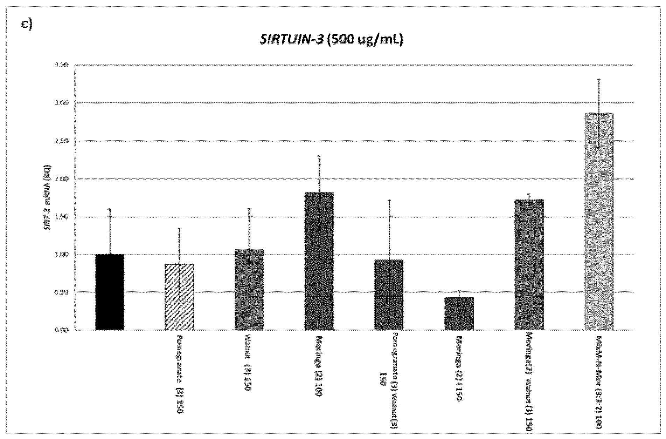

Figures 5a), b), c) show bar graphs representing SIRT-3 gene expression in human NCTC2544 keratinocytes as determined by RT-PCR. NCTC2544 cells were treated for 24 hours at 37°C, 5% CO₂, with: basal medium containing 2.5% FBS (vehicle); the vegetal components Pomegranate; Walnut; Moringa, singly and as mixture of two, and as mixture of three (basic mixture). The combination of three basic components was tested at 10 µg/mL (a), 100 µg/mL (b) and 500µg/mL (c), respectively, and the active vegetal components were also tested at a concentration reflecting that in the final combination. The values are the average of 2 duplicate experiments.

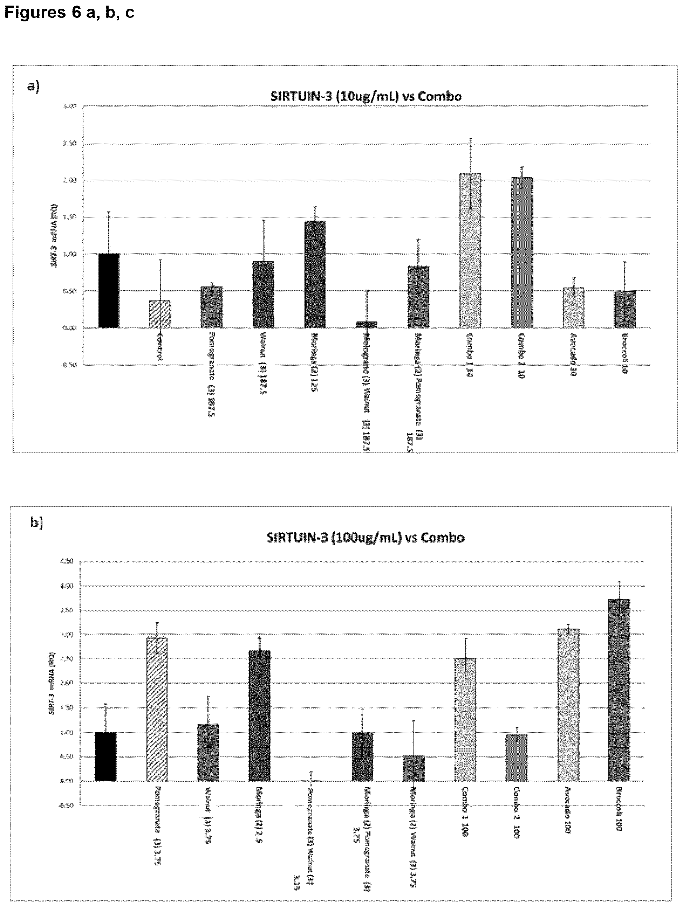

Figures 6a), b), c) show bar graphs representing SIRT-3 gene expression in human NCTC2544 keratinocytes as determined by RT-PCR. NCTC2544 cells were treated for 24 hours at 37°C, 5% CO₂, with: basal medium containing 2.5% FBS (vehicle); Pomegranate; Walnut; Moringa, singly and as mixture of two, Avocado; and Broccoli singly, and the compositions Combo 1, Combo 2 of the invention. The combination compositions having a ratio of 3:3:2:2 were tested at 10µg/mL (a), 100 µg/mL (b) and 500µg/mL (c), respectively, and the active vegetal components were also tested at a concentration reflecting that in the final combination. The values are the average of 2 duplicate experiments.

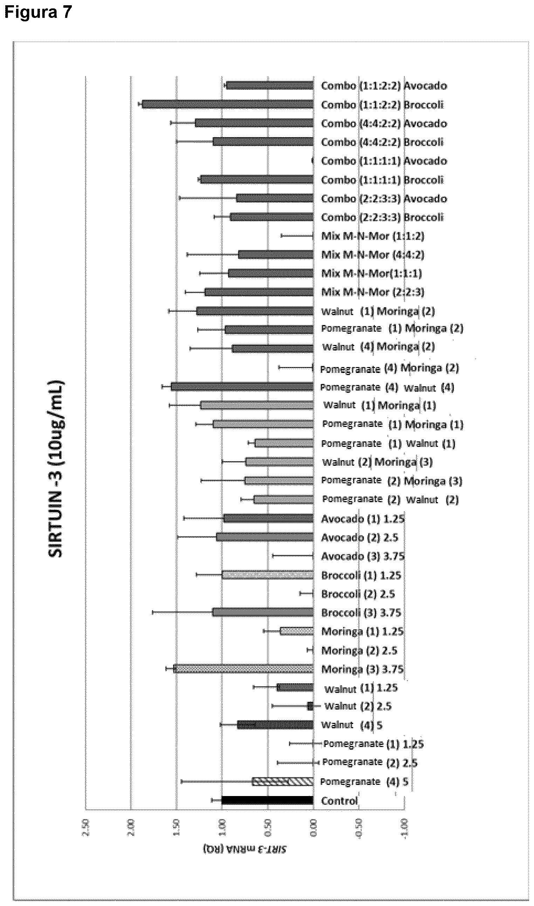

Figure 7 shows bar graphs representing *SIRT-3* gene expression in human NCTC2544 keratinocytes as determined by RT-PCR. NCTC2544 cells were treated for 24 hours at 37°C, 5% CO₂, with: basal medium containing 2.5% FBS (vehicle); the single vegetal components Pomegranate; Walnut; Moringa, two-component mixtures thereof, three vegetal component basic mixture, and compositions of the invention (Combo) comprising the three-component basic mixture and one of Avocado or Broccoli in various proportions. The combinations (2:2:3:3; 1:1:1:1; 4:4:2:2; 1:1:2:2) were tested at 10µg/mL, and the active vegetal components were also tested at a concentration reflecting that in the final combination. The values are the result of 2 duplicate experiments.

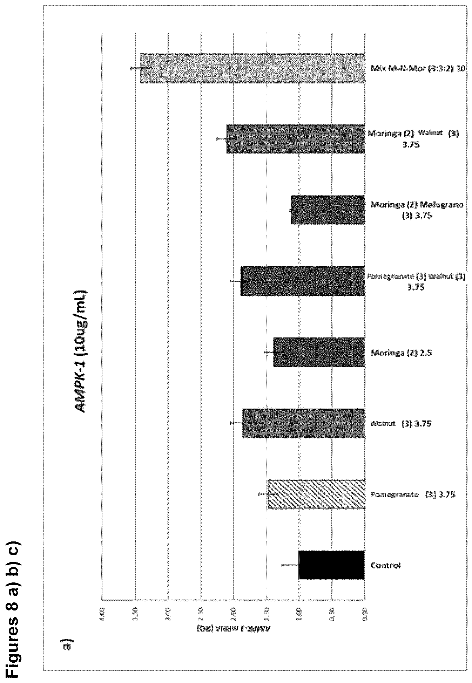

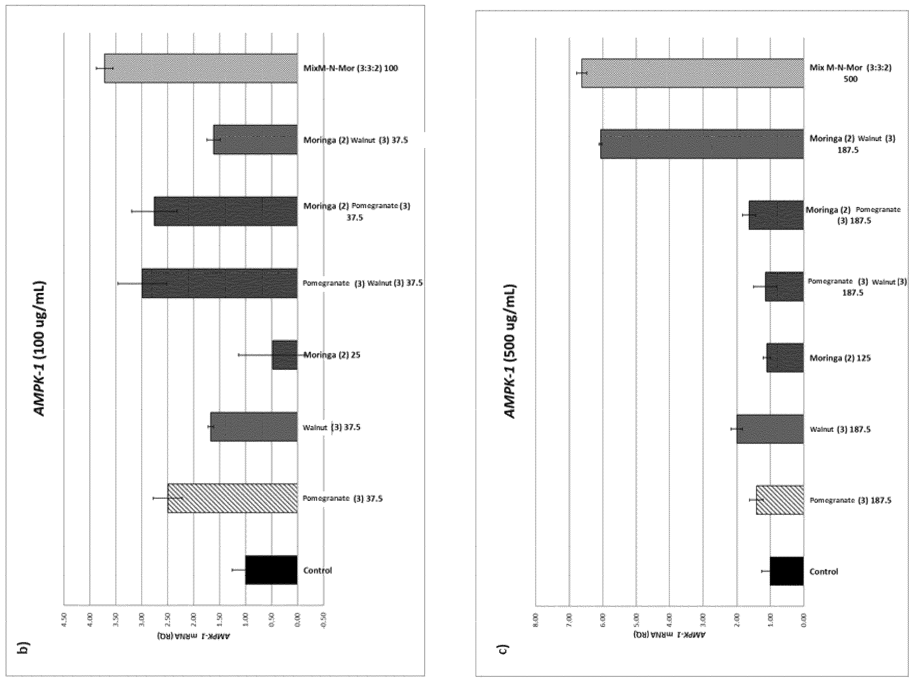

Figures 8a), b), c) show bar graphs related to SIRT-1 gene expression in human NCTC2544 keratinocytes as determined by RT-PCR. NCTC2544 cells were treated for 24 hours at 37°C, 5% CO₂, with: basal medium containing 2.5% FBS (vehicle); the vegetal components Pomegranate; Walnut; Moringa singly, and as mixture of two, and as mixture of three (basic mixture). The combination of three components was tested at 10µg/mL (a), 100 µg/mL (b) and 500µg/mL (c), respectively, and the active vegetal components were also tested at a concentration reflecting that in the final combination. The values are the average of 2 duplicate experiments.

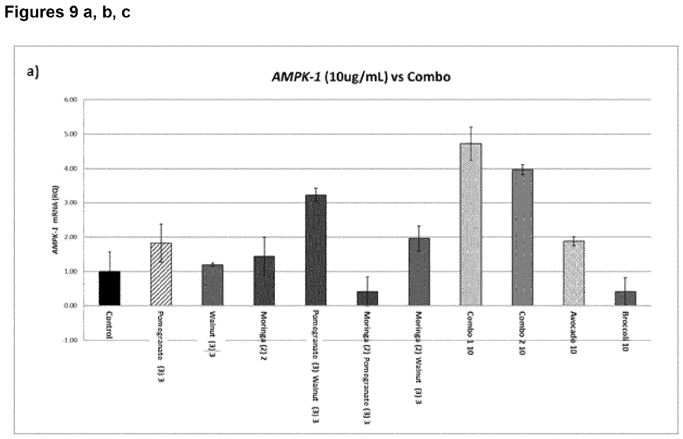

Figures 9a), b), c) show bar graphs related to AMPK-1 gene expression in human NCTC2544 keratinocytes as determined by RT-PCR. NCTC2544 cells were treated for 24 hours at 37°C, 5% CO₂, with: basal medium containing 2.5% FBS (vehicle); Pomegranate; Walnut; Moringa singly, and as mixture of two; Avocado; and Broccoli singly, and compositions Combo 1; Combo 2 of the invention. The combination compositions having a ratio of 3:3:2:2 were tested at 10µg/mL (a), 100 µg/mL (b) and 500µg/mL (c), respectively, and the active vegetal components were also tested at a concentration reflecting that in the final combination. The values are the average of 2 duplicate experiments.

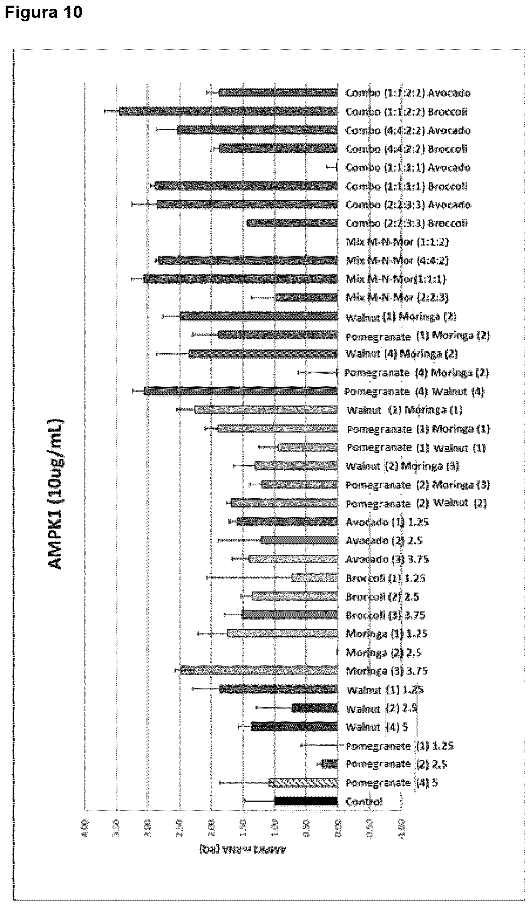

Figure 10 shows bar graphs related to *AMPK1* gene expression in human NCTC2544 keratinocytes as determined by RT-PCR. NCTC2544 cells were treated for 24 hours at 37°C, 5% CO₂, with: basal medium containing 2.5% FBS (Vehicle); the single vegetal components Pomegranate; Walnut; Moringa; mixtures of two vegetal components, basic mixture of the three vegetal components, and compositions of the invention (Combo) comprising the three-component basic mixture and one of Avocado or Broccoli, in various proportions.
   The combinations (2:2:3:3; 1:1:1:1; 4:4:2:2; 1:1:2:2) were tested at 10 µg/mL, and the active vegetal components were also tested at a concentration reflecting that in the final combination. The values are the result of 2 duplicate experiments.

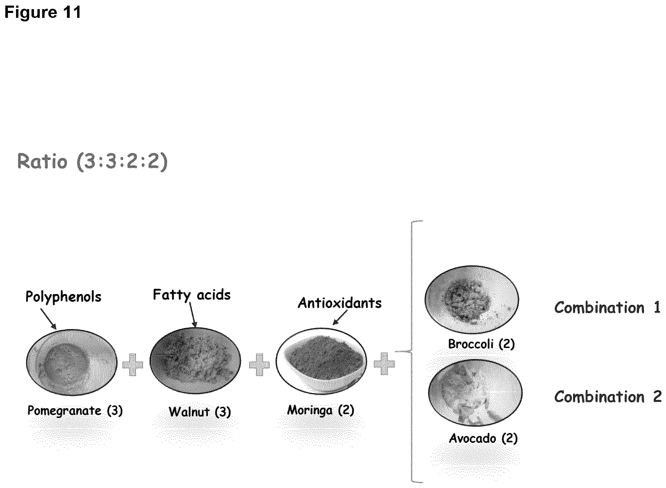

Figure 11 illustrates combinations of the basic mixture of the three nutritional components of vegetal origin with the additional vegetal component selected from Avocado and Broccoli in shredded form according to embodiments of the invention.

### Detailed Description of the Invention

The present invention originates from the finding that by combining, in selected ratios, a basic vegetal nutritional component comprising a mixture of Pomegranate, Walnut and Moringa with one selected vegetal nutritional component selected from Avocado and Broccoli, a synergistic activation of sirtuins, in particular *SIRT-1, SIRT-3, and AMPK-1,* recognized markers of the longevity of an individual, is obtained.

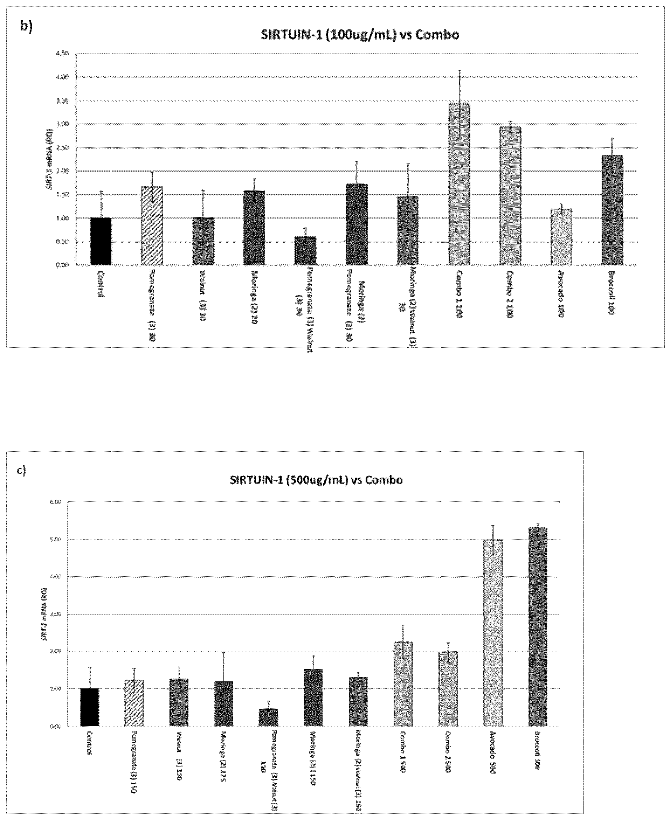

This effect is greater than that found using the same vegetal components separately or combined two by two, as evidenced for example by the data shown in Figures 3, 4, 6, 7, 9, 10.

The synergistic action on sirtuins of the nutritional vegetal component mixtures, in the specific proportions identified by the inventors, allows to slow down the physiological biochemical processes of aging of the human body, thus increasing the longevity of the individual.

In accordance with a first aspect of the invention, a composition is therefore provided in accordance with the appended claim 1.

The compositions of the invention comprise three basic nutritional components of vegetal origin which are advantageously mixed together in predefined ratios.

The three basic vegetal nutritional components are Pomegranate, Walnut and Moringa. These components are present or mixed in the composition in quantitative ratios of 2.7-3.3 : 2.7-3.3 : 1.7-2.3 or 2.8-3.2 : 2.8-3,2 : 1.8-2.2, or 2.9-3.1 : 2.9-3.1 : 1.9-2.1, preferably 3:3:2.

Typically, the ratios between the components are ratios by weight, or by weight and volume, between the various components, as described herein.

The basic nutritional components Pomegranate, Walnut and Moringa, present in the aforementioned ratios, are combined with one further selected vegetal nutritional component/fruit selected from Avocado and Broccoli.

The further vegetal component/fruit is present in a ratio ranging from 1.7 to 2.3, or from 1.8 to 2.2, or from 1.9 to 2.1, in particular equal to 2, with respect to the basic components.

Advantageously, in the composition, the vegetal nutritional components and/or fruit are in a shredded, blended form or as a vegetal pulp/puree form.

As shown in Figure 3, the preferred proportions between the four components (three of the basic mixture plus a selected one) of the composition is 3:3:2:2.

The combination of the mixtures of selected nutritional components in the selected quantitative ratios described herein results in an unexpected synergistic activation effect on SIRT-1, SIRT-3 and AMPK-1 which is the basis of the increase in longevity in the treated cells.

This finding was experimentally demonstrated by the inventors through some tests in which the three basic nutritional components of the invention, Pomegranate, Walnut and Moringa, were tested as single components, in pairs and as a mixture, specifically in a ratio of 3:3:2, respectively, as shown in Figure 2, Figure 5, Figure 8. In particular, the basic mixtures of the three nutritional components in a ratio of 3:3:2 have shown, when tested at a concentration of 10-100 and 500 µg/mL, that the effect found is superior to the components tested separately or in a mixture of two, as evidenced by the data shown in Figure 2, Figure 5, Figure 8. The most marked effect is at doses of 10 and 100 µg/mL.

In particular, the combinations of the basic nutritional components in a ratio of 3:3:2, combined in such ratio with Broccoli and/or Avocado (3:3:3:2) have shown, when tested at a concentration of 10-100 and 500 µg/mL, that the effect found is superior to the components tested separately or in pairs, as evidenced by the data shown in Figure 3, Figure 6, Figure 9. The most marked effect is at doses of 10 and 100 µg/mL.

Such an effect is also superior to that of combinations 2:2:3:3; 1:1:1:1; 4:4:2:2; 1:1:2:2, as evidenced by the data shown in Figure 4, Figure 7, Figure 10, at a concentration of 10 µg/mL.

According to certain aspects, the invention provides the composition as described herein for use in increasing the longevity of an individual.

The individual can be a mammal, and specifically a human being.

Embodiments of the composition of the invention are defined in the appended dependent claims 2-9.

The biologically active components present in the composition are for use in the nutraceutical, nutritional, cosmetic, or aesthetic medicine fields.

According to some aspects of the invention, it is possible to treat a population of subjects to maintain the main biochemical parameters at a physiological level or to increase life expectancy.

According to other aspects, the invention provides a composition as described herein to reduce the risks and/or prevent the development of diseases that can reduce an individual's life expectancy or to keep the main biological parameters of a human being within the physiological limits.

In accordance with other aspects, the composition of the invention finds application in topical use.

According to these aspects, the invention relates to the topical, non-therapeutic use of a composition as described herein, containing the combination of components described herein, in the treatment of aesthetic skin damage or skin aging caused by external agents, in particular by exposure to ultraviolet radiation.

In accordance with these aspects, the composition of the invention finds application in the cosmetic treatment of skin aging signs resulting from exposure to ultraviolet radiation, typically sun rays.

Typically, the composition of the invention is used in the prevention or non-therapeutic treatment of photo-aging or skin aging.

Furthermore, the composition of the invention can be used in preventing or treating those signs of skin aging that are referrable to repeated exposure over time to ultraviolet radiation such as, for example, skin corrugation, roughness, thickening of the skin, dehydration and wrinkling of the skin of the body, and in particular of the face.

The vegetal components, including the fruits of the composition of the invention, used in the method described herein, are sources rich in biogenic compounds and have very specific and complementary characteristics.

These components and other aspects of the composition of the invention are described in detail below.

One of the nutritional components is based on pomegranate, or a part or extract thereof. Pomegranate (*Punica* granatum *L*.) is a plant whose fruit, known as pomegranate, is a round or slightly elongated berry with a hard skin. The fruit has several robust internal partitions that perform the function of placentation for the seeds, called grains or arils, separated by a membrane called sarcotesta. In some varieties the seeds, red in colour, are surrounded by a translucent pulp with a colour from white to ruby red. The fruit bears in the apical position (opposite to the petiole) a characteristic robust crown of four to five pieces, which are residues of the floral calyx. This plant, and in particular its fruit, has a high antioxidant activity due to the relevant content of anthocyanins, ellagic acid derivatives and hydrolysable tannins. Pomegranate juice has three times the antioxidant activity of red wine and green tea. Preferably, the pomegranate fruit is used. Suitable extracts are prepared from the fruit, in particular from rind or seeds or arils thereof.

One of the nutritional components of the composition is based on walnuts.

Walnuts are the fruit of a tree of the genus *Juglans L,* in particular of the *Juglans nigra* species. Walnuts are known for their nutritional properties. Walnuts have a lipid profile that has been linked to a wide range of biological properties and health-promoting effects. In addition to essential fatty acids, walnuts contain a variety of other bioactive compounds such as vitamin E and polyphenols. Walnuts possess well known antioxidant and anti-inflammatory bioactivities and several studies have assessed the potential role of walnuts against initiation and progression of disease, including cancer, cardiovascular and neurodegenerative diseases. In one embodiment, the Californian variant *Juglans nigra* is used.

For the preparation of the composition, it is possible to use the seed contained in the drupe, covered by a woody endocarp, or a portion thereof, or an extract thereof using an extraction technique as described herein.

One of the nutritional components of the composition is based on Moringa (*Moringa oleifera Lam.*), or a portion or extract thereof.

For the preparation of the composition, it is possible to use moringa leaves, fruits, seeds, flowers and roots, or portions thereof. In particular, from leaves, fruits and seeds it is possible to extract oil, from flowers and roots it is possible to obtain an extract using an extraction technique as described herein.

The fruits possess anti-biocidal, antitumor, antioxidant, anti-inflammatory, cardioprotective, hepatoprotective, neuroprotective, tissue-protective activities.

Vegetal tissues have an important variety of alkaloids and sterols, polyphenols and phenolic acids, fatty acids, flavanoids and glycosides, flavanol, glucosinolate and isothiocyanate, terpenes and anthocyanin.

One of the nutritional components is based on avocado, or a portion or extract thereof.

Avocado (*Persea americana Mill*), a native American vegetal species, is very rich in polyphenols, carotenoids, tocopherols and phytosterols, which provide it with antimicrobial, anti-inflammatory, antitumor, antidiabetic and antihypertensive biological activities.

The fruit is a pear-shaped drupe, and it has a large, 3-5 cm in diameter, central seed. The pulp is yellow-green or pale yellow, the epicarp or peel can be green or aubergine, smooth or wrinkled.

The fruit, without seed, is preferably used. For the preparation of the composition, it is possible to use the fruit as it is, or a portion thereof, or an extract thereof, by resorting to an extraction technique as described herein.

One of the nutritional components of the composition is based on broccoli or a portion or extract thereof.

Broccoli belongs to the Brassica oleracea species, var. italica, an edible green plant in the family Brassicaceae, genus *Brassica,* whose not yet ripe inflorescences are edible and eaten. Broccoli is also known as cabbage-broccoli, as it belongs to the category of the same name. Broccoli contains vitamin C and β-carotene and polyphenols, *sulforaphane* and chlorophyll.

Methods for preparing the compositions of the invention are described herein.

According to some embodiments, the following parts/portions of vegetable/fruit are used to make the nutritional composition described herein:
- Pomegranate, part used: seeds or arils;
- Walnut, part used: kernels;
- Moringa, part used: leaf extract, in the form of a dry extract obtained using traditional extraction techniques;
- Avocado, part used: fruit pulp;
- Broccoli, part used: curd.

One embodiment of the method for preparing the nutritional composition was standardized as described herein. All fresh matrices were washed, cleaned, and cut into small pieces. The walnuts were separated from the inedible part. The avocado stone was removed, and the pomegranate seeds or arils separated from the peel. All the ingredients were mixed by a vertical food processor (R8 Robot Coupe model, Bologna, Italy). After mixing, the residual pomegranate juice was filtered using a common sieve. The moringa powder was dissolved with water, preferably distilled water, in a ratio of 1:9, w/v, and mixed manually. Preliminarily, different combinations were prepared using different ingredients, combining a different number of ingredients, and varying the ratio of ingredients.

The 5 prepared combinations contain three common ingredients/components: Pomegranate, Walnut, Moringa and a fourth variable component/ingredient: Broccoli, Avocado.

The final preparations comprised ingredients and ratios as follows:
Preparation 1: Pomegranate, Walnut, Moringa, Broccoli (3: 3: 2: 2, v / w / v / w)
Preparation 2: Pomegranate, Walnut, Moringa, Avocado (3: 3: 2: 2, v / w / v / w)
Where v means volume and w means weight.

The composition of the invention can be a diet or nutritional supplement, a drug.

According to certain embodiments, the composition of the invention further comprises one or more active substances, such as vitamins, minerals, micronutrients, and other active substances, and optionally edible vehicle and/or excipients.

Within the scope of the invention, the term "edible or edible" means a physiologically acceptable substance that can be taken by a human being and falls within a conventional eating regime or diet.

According to some embodiments, the composition for oral administration is contained in a functional food, a nutraceutical composition, a dietary product, a supplement, or a nutritional product.

Nutraceutical product means a product isolated or purified from edible substances. A nutraceutical is such when it is shown that it has a physiological benefit or provides protection against a physiological inconvenience or disorder.

Food supplement means a source of one or more nutrients (vitamins, minerals, amino acids, lipid substances, including unsaturated and polyunsaturated fatty acids, vegetal extracts) made in pre-dosed forms and intended to supplement the common diet.

Enriched food, on the other hand, means a commonly used food to which nutrients have been added.

Food for special medical purposes refers to preparations intended for those who, due to their condition, have limited or impaired ability to take, digest, absorb or metabolize normal food.

The amount administered and the frequency of administration of the composition will depend on the nature and severity of the condition to be treated.

In some embodiments, the composition of the invention comprises a physiologically and/or pharmaceutically acceptable vehicle, diluent or excipient.

Typically, the physiologically acceptable vehicle of the composition of the invention is an excipient or diluent suitable for topical application and/or for oral administration. Within the scope of the present invention, the term "vehicle" refers to the set of inert ingredients or excipients, which may be present in the composition of the invention. Any vehicle and/or excipient suitable for the form of preparation desired for administration is contemplated in the uses of the vegetal extract, or active ingredients present therein, described herein.

In certain embodiments, the composition of the invention contains further components of vegetal origin that are biologically active and substantially free of side effects, when administered orally or locally.

The compositions of the present invention can be used as such or used to form edible vehicles through the use of other ingredients, such as excipients, technological adjuvants selected according to the intended use (systemic or topical administration) or to satisfy particular regulatory requirements (e.g. food rather than food for special medical purposes).

Advantageously, the composition is mainly in a form for oral administration.

The compositions for oral administration can be in a solid or semi-liquid form.

For example, when it is in a solid form it can be presented as powder, granules, tablet, capsule, bars or as a lyophilized product; when it is in a semi-liquid form it can be presented as smoothies, gel or shake.

The composition of the invention can be treated using water removal techniques, such as freeze drying, drying, spray-drying, etc. The composition can also be subjected to heat treatment aimed at reducing the microbial load, such as pasteurization, sterilization, tantalization, etc.

For example, freeze-drying of the composition includes the treatment of the nutritional vegetal components through the following steps and conditions:
Freezing: 50°C, 2 h
Main lyophilization: -40°C for 1 h, -30°C for 1 h, -20°C for 1 h, -10°C for 3 h, 0°C overnight, +10°C for 1 h;
Final lyophilization: 20°C for 4 h
Sample freezing temperature: -30°C
Main drying working pressure: 0.128 mbar
Final drying working pressure: 23.4 mbar
Safety Pressure: 0.380 mbar
Advantageously, the composition described herein, in particular in a semi-liquid form, can be pasteurized by adopting traditional methods.

For example, pasteurization requires the five components to be blended to form smoothies and then transferred to containers, such as glass bottles, and immersed in boiling water, typically in a thermostatic bath set at 100°C. When the temperature inside the smoothies has reached 80°C, this temperature is maintained for 5 minutes before removing the pasteurized compositions/samples.

In some embodiments, the composition is in a topical form and is applied to the skin.

In some embodiments, the route of administration of the composition of the invention is therefore topical administration.

In these cases, the composition of the invention can be in the form of emulsion, cream, liniment, ointment, lotion, mask, patch.

In applications in the cosmetic/aesthetic field, for example in preventing or treating photo-aging, it is possible to apply a cosmetically active amount of the composition of the invention on the affected skin area, one or more times a day, conveniently for a period of at least 2-3 months.

According to another aspect of the invention, a cosmetic treatment method is provided, comprising application to the skin of an effective amount of a composition of the type described above.

The composition for topical application can be in a solid, semi-solid or fluid form. Suitable formulations in solid form include pastes, creams, gels, liniments, ointments, masks, muds.

In some embodiments, the compositions of the invention may comprise excipients commonly used in the formulation of cosmetic or pharmaceutical preparations, for local use, such as preservatives, bactericidal agents, stabilizers, emulsifiers, buffers, humectants, colouring agents, and other excipients commonly used in cosmetic/pharmaceutical preparation techniques.

### EXAMPLE 1

### Composition in the form of powders for extemporaneous liquid dispersion stored in sachet

| **Substance** | **mg/dose** |
|---|---|
| Combination 1 or Combination 2 | 850 |
| Maltodextrins | 450-1000 |
| Erythritol (E968) | 100 |
| Citric Acid (E330) | q.s. |
| Silicon dioxide | 15 |
| Flavours | q.s. |
| Sucralose | q.s. |

### EXAMPLE 2

### Composition in the form of TABLETS

| **Substance** | **mg per tablet** |
|---|---|
| Combination 1 or Combination 2 | 150 |
| Acerolad.e | 50 |
| Beetroot d.e | 50 |
| Astragalus d.e. | 30 |
| Quercetin | 30 |
| 50% Vitamin E acetate (carrier: silicon dioxide) | 6 |
| 10% Lycopene by direct compression | 10 |
| Vitamin B3 (Nicotinamide) | 16 |
| Microcrystalline cellulose | 30-150 |
| Calcium phosphate dibasic dihydrate | 30-200 |
| Hydroxypropyl cellulose | 10-100 |
| Silicon dioxide | 7-10 |
| Vegetal magnesium stearate | 7-10 |

### EXAMPLE 3

### Composition in the form of an extemporaneous powder for Umami flavour Smoothie

| Substance | Amount/unit |
|---|---|
| Combination 1 or Combination 2 | 1-25 grams |
| Flavour | 100-1000 mg |
| Salt (sodium chloride) | 5-1000 mg |
| Monosodium glutamate | 1-200 mg |
| Arabic gum | 10-1000 mg |
| Guar gum | 10- 600 mg |
| Inulin | 100-1000 mg |
| Rosemary extract | 1-50 mg |
| Hydrolysed wheat proteins | 100-2000 mg |
| Silicon dioxide | 10-200 mg |

Packaged in a protective atmosphere.

### EXAMPLE 4

### Composition in the form of an extemporaneous powder with chocolate flavour

| **Substance** | **Amount/unit** |
|---|---|
| Combination 1 or Combination 2 | 1-15 grams |
| Chocolate flavour | 100-1000 mg |
| Vanilla flavour | 1-200 mg |
| Sugar (saccharose) | 5-1000 mg |
| Maltodextrins | 50-1000 mg |
| Walnuts in pieces | 1-200 mg |
| Arabic gum | 10-1000 mg |
| Guar gum | 10- 600 mg |
| Inulin | 100-1000 mg |
| Vitamin C (ascorbic acid and sodium ascorbate) | 10-60 mg |
| 18% Low-fat cocoa powder (low-fat cocoa, lecithin). | 1-50 mg |
| Hydrolysed wheat proteins | 100-2000 mg |
| Milk proteins | 10-500 mg |
| Silicon dioxide | 10-200 mg |
| Soybean oil powder (soybean oil, glucose syrup, milk protein, natural flavour) | 10-500 |

Packaged in a protective atmosphere.

### EXAMPLE 5

### TABLETS

| **Substance** | **mg per tablet** |
|---|---|
| Combination 1 or Combination 2 | 150 |
| Whitamnia somnifera d.e . | 50 |
| L-Carnitine by direct compression | 50 |
| Carnosine | 50 |
| Lactoferrin | 50 |
| Microcrystalline cellulose | 100 |
| Calcium phosphate | 150 |
| Silicon dioxide | 5 |
| Vegetal magnesium stearate | 8 |

### EXAMPLE 6

Composition for topical application

| **Compound/INCI** | **%** |
|---|---|
| AQUA | 74.05 |
| HYDROXYACETOPHENONE | 0.30 |
| ACRYLATES C10-30 ALKYL | |
| ACRYLATES CROSSPOLYMER | 0.25 |
| CARBOMER | 0.20 |
| GLYCERIN | 3.00 |
| DISODIUM EDTA | 0.10 |
| CAPRYLIC/CAPRIC TRIGLYCERIDE | 10.00 |
| DICAPRYLYL CARBONATE | 5,00 |
| Combination 1 or Combination 2 | 5.00 |
| LECITHIN | 0.25 |
| AQUA, SODIUM HYDROXIDE | 0.35 |
| 1,2-HEXANEDIOL | 1.50 |

### EXAMPLE 7

Composition for topical application

| Compound/INCI | % |
|---|---|
| AQUA | 69.05 |
| HYDROXYACETOPHENONE | 0.30 |
| ACRYLATES C10-30 ALKYL | |
| ACRYLATES CROSSPOLYMER | 0.25 |
| CARBOMER | 0.20 |
| GLYCERIN | 3.00 |
| DISODIUM EDTA | 0.10 |
| CAPRYLIC/CAPRIC TRIGLYCERIDE | 10.00 |
| DICAPRYLYL CARBONATE | 5.00 |
| Combination 1 or Combination 2 | 10.00 |
| LECITHIN | 0.25 |
| AQUA, SODIUM HYDROXIDE | 0.35 |
| 1,2-HEXANEDIOL | 1.50 |

### EXAMPLE 8

Test to verify the synergistic effect of combinations 1 and 2 containing Pomegranate, Walnut, Moringa (Basic Composition) combined with Avocado (Composition 1) or Broccoli (Composition 2) in selected ratios.

### Aim

The object of the project is to experimentally verify the existence of a synergistic effect on Sirtuin 1, Sirtuin 3 and AMPK1 by the following combinations (Combo): Combinations of Pomegranate, Walnut, Moringa (Basic Mixture) with Avocado or Broccoli (alternative components) in the following ratios:
- (3:3:3.2)
- (2:2:3:3)
- (1:1:1:1)
- (4:4:2:2)
- (1:1:2:2)

The term "Combo" means Combination or Combination Composition; in particular Combo 1 means a combination of the basic mixture of Pomegranate, Walnut, Moringa with Broccoli; Combo 2 means a combination of the basic mixture of Pomegranate, Walnut, Moringa with Avocado

### MATERIALS

### Vegetal components tested

| ID | TESTED CONC. | STORAGE |
|---|---|---|
| Basic composition - Vegetal components | | |
| Pomegranate | 1.25-2.5-3-3.75-5-30-37.5-150-187.5 µg/mL | 4°C |
| Walnut | 1.25-2.5-3-3.75-5-30-37.5-150-187.5 µg/mL | 4°C |
| Moringa | 1.25-2-2.5-3.75-20-25-100-125 µg/mL | 4°C |
| | | |
| Vegetal component to be added to the basic comp. | | |
| Avocado | 1.25-2.5-3-10-100-500 µg/mL | 4°C |
| Broccoli | 1.25-2.5-3-10-100-500 µg/mL | 4°C |
| | | |
| Basic comp. + additional component combinations | | |
| Combo 1 | 10-100 and 500 µg/mL | 4°C |
| Combo 2 | 10-100 and 500 µg/mL | 4°C |
| Combo (2:2:3:3) Broccoli | 10 µg/mL | 4°C |
| Combo (2:2:3:3) Avocado | 10 µg/mL | 4°C |
| | | |
| Combo (1:1:1:1) Broccoli | 10 µg/mL | 4°C |
| Combo (1:1:1:1) Avocado | 10 µg/mL | 4°C |
| Combo (4:4:2:2) Broccoli | 10 µg/mL | 4°C |
| Combo (4:4:2:2) Avocado | 10 µg/mL | 4°C |
| Combo (1:1:2:2) Broccoli | 10 µg/mL | 4°C |
| Combo (1:1:2:2) Avocado | 10 µg/mL | 4°C |

Pomegranate, Walnuts and Moringa were tested at the same ratio in combination and in a mix of all three. In the combination (Combo) of the basic component with the additional component, the components were tested at the concentration corresponding to the amount in the final mixture.

### Reagents and instruments used

| **REAGENTS** | **SUPPLIER** |
|---|---|
| Human keratinocyte cell line NCTC 2544 | HL97002, Istituto Zooprofilattico Brescia |
| RPMI medium | Sigma Aldrich |
| Fetal bovine serum (FBS) | Sigma Aldrich |
| 2 mM L-glutamine | Sigma Aldrich |
| 1% penicillin (10,000 U/mL)/streptomycin (10,000 µg/mL) | Sigma Aldrich |
| Trypsin Edta | Sigma Aldrich |
| Gentamycin | Sigma Aldrich |
| Tri reagent Solution | Thermo Fisher, AM9738 |
| Ethidium bromide solution (10 mg/mL, for molecular biology, aqueous solution) | SIGMA, E1510 |
| Gel Loading Buffer RNAse, none detected | SIGMA, G2526 |
| PRIME SCRIPT RT reagent kit (Perfect Real time) 200 rxn | TAKARA, RR037A |
| PreMix Ex Taq | TAKARA, RR039A |
| Chloroform | SIGMA, 366919-1L |
| Isopropanol | SIGMA, I9516-500 ml |
| TaqMan^{®} Gene Expression Assays for GAPDH Hs99999905_m1 | APPLIED BYOSISTEMS, 4331182 |
| TaqMan^{®} Gene Expression Assays for SIRT-1 HS01009006_M1 (SIRT-1) | APPLIED BYOSISTEMS, 4331182 |
| TaqMan^{®} Gene Expression Assays for SIRT-3 HS00953477_M1 | APPLIED BYOSISTEMS, 4331182 |
| TaqMan^{®} Gene Expression Assays for AMPK-1 HS00234508_M1 | APPLIED BYOSISTEMS, 4331182 |

| INSTRUMENTS | SUPPLIER |
|---|---|
| Inverted Phase Contrast Microscope (Mod: DMIL) | Leica |
| Laminar flow cabinet (Mod: Gemini) + UV lamp with anti-reflex equipment | Steril Manifacturing Division |
| HeraCell CO2 incubator (Mod:150 ADV) | Thermo Scientific |
| 15 L digital water bath from +5°C to + 100°C (Mod: Swbd1, BS-SWB2D) | Stuart |
| 85°C horizontal freezer ULT130, 120 L (Mod: Labfrost, MME-TE21140) | Elcold |
| Bürker counting chamber w/clamps (DI-DA-443/3) | Carlo Erba |
| Balance (Mod. AM100) | Mettler |
| RT PCR MX300P | Stratagene |
| Vortex | Arhos160-PBI International |

### Biological Model

### Human keratinocytes NCTC2544

The immortalized line of human keratinocytes NCTC 2544 (Perry V.P. et al., 1957) kept in culture in sterile flasks (25 cm³), incubated at 37°C in a humid atmosphere with 5% CO₂ in RPMI-1640 culture medium supplemented with 10% fetal bovine serum (FBS), 2mM glutamine, 1% penicillin and streptomycin, and 0.1% gentamycin, is used.
The 1:3 split is performed every 2 days, upon reaching the monolayer, by washing with 1X PBS (phosphate buffer without Ca²⁺ and Mg²⁺) and detaching the cells with a trypsin-EDTA solution at 37°C for 3 minutes. Cells were seeded at a density of 1×10⁶ cells per well in 12-well plates for qRT-PCR.

| | | |
|---|---|---|
| **ICLC CATALOG CODE** | HL97002 | |
| **DEPOSITOR** | Prof. M. Ferro, DIMES, General Pathology, University of Genoa, Italy | |
| **BIBLIOGRAPHIC REFERENCES** | | • Arch Dermatol Res 1976; 256 (3): 255-260-PMID: 990102 |
| | | • Arch Dermatol Res 1976; 261 (1): 27-31 |

### Controls

NEGATIVE CONTROL: Untreated cells in RPMI medium under the same culture conditions.

### Methods

### LONGEVITY TEST

24 hours after seeding on 12-well plates, the 80% confluent cells NCTC2544 were exposed to different concentrations (see table) of single or combined vegetal compounds. RNA for qRT-PCR analysis was extracted 24 hours after treatment using the Tri Reagent (Sigma Aldrich) described by Chomczynski and Mackey (Chomczynski P, Mackey K. 1995), and the cDNA was then synthesized from 2 µg of RNA template in a reaction volume of 20 µL, using the PrimeScript RT-PCR kit (Takara, Japan). The cDNA was amplified and detected by the Stratagene Mx3000P system in Real Time PCR (Agilent Technologies Italia S.p.A., Milano, Italy). Amplification of cDNA from NCTC2544 cells was carried out using the following Taqman probes: HS00272166_M1 (AMPK-β1), HS00953477_M1 (SIRT-3), HS01009006_M1 (SIRT-1) and Hs999999 m1 (human glyceraldehyde-3-phosphate dehydrogenase, GAPDH). GAPDH was used as housekeeping gene. PCR amplifications were performed in a total volume of 20 µL. The reaction mixture contained 10 µL of 2X Premix Ex Taq (Takara, Japan), 1 µL of 20 × TaqMan gene expression assay, 0.4 µL of RoX Reference Dye II (Takara, Japan), 4.6 µL of water and 4 µL of DNA. PCR conditions were as follows: 95°C for 30 seconds, followed by 40 cycles at 95°C for 5 seconds, 60°C for 20 seconds. PCR reactions were performed in duplicate using a PCR MX3000p machine (Stratagene, La Jolla, CA).

The cycle threshold ΔCt (Vigetti et al., 2008) was used to calculate the relative abundance in the expression of each gene.

### RESULTS

Sirtuins are proteins involved in many human pathways and are gaining national attention due to their role as "longevity proteins" that can extend and improve human life (3).

As highlighted in the accompanying Figure 1, sirtuins play a role in some of the main human biological/metabolic processes (4).

Once activated, they interact with other molecular longevity targets such as, for example, AMPK (AMP activated protein kinase (3)). AMPK activation leads to the regulation of numerous signaling pathways involved in senescence and aging, such as those involving p53, mTOR and NFκB (5).

At the same time, the activation of AMPK increases the level of NAD+ (also through increasing the level and activity of NAMPT) thereby increasing SIRT1 activity (6). Sirtuins, particularly SIRT-1, also regulate canonical TGF-β signaling (7).

### Description

The combinations of vegetal components reported in the Table under Materials, were tested for their *in vitro* activity as longevity stimulator by qRT-PCR. The main genes involved in aging are studied: *SIRT-1, SIRT-3* and *AMPK-1.*

In order to evaluate the synergistic activity of the combination of vegetal components mixed in different ratios, the vegetal components were analysed as a single component or in different combinations.

The vegetal components Pomegranate, Walnut and Moringa were tested for their activity as single components, in pairs and as a mixture (basic mixture), respectively, in the following ratios: 3:3:2; 2:2:3, 1:1:1, 4:4:2 and 1:1:2, as shown in Figures 2, 5, 8.

The single additional vegetal components, Avocado and Broccoli, were tested:
in a ratio of 2 with the 3:3:2 combination of Pomegranate, Walnut and Moringa;
in a ratio of 3 with the 2:2:3 combination of Pomegranate, Walnut and Moringa;
in a ratio of 1 with the 1:1:1 combination of Pomegranate, Walnut and Moringa;
in a ratio of 2 with the 4:4:2 combination of Pomegranate, Walnut and Moringa;
in a ratio of 1 with the 1:1:2 combination of Pomegranate, Walnut and Moringa.

The term Combo, where mentioned, is to be understood as a combination or combination composition, according to the invention. The term paired means combined.

Pomegranate, Walnut and Moringa were tested for their activity as a single compound, paired, and mixed in a ratio of 3:3:2, respectively, as reported in Figure 2, Figure 5, Figure 8, respectively.

The 3:3:2 combinations showed a synergistic effect on SIRT-1, SIRT-3 and AMPK-1, when tested at a final concentration of 10-100 and 500µg/mL, respectively, and this effect was superior to that of compounds tested separately or combined (Figure 2, Figure 5, Figure 8). The effect was more apparent at 10 and 100 µg/mL.

Furthermore, the Pomegranate, Walnut and Moringa mixture, with a ratio of 3:3:2 was tested in combination with other fruits and vegetables in a ratio of 2: Avocado/Broccoli. Fruit and vegetables were tested, also in this case, as a single component taking into account their amount in the final combination.

Figure 3 shows the effect of the combinations (Combo 1, 2) on SIRT-1. Both combinations showed a synergistic effect on SIRT-1 activation, when tested at 10 µg/mL, and their effect is superior to that of single compounds. This also applies to combination 1, 2 at 100 µg/mL. No synergistic effect was reported at 500 µg /mL.

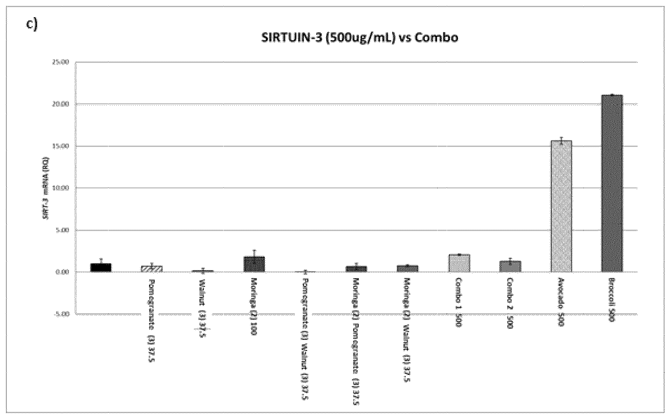

As illustrated in Figure 6, both combinations also showed a synergistic effect on SIRT-3 activation, when tested at 10 µg/mL, and their effect is superior to that of single compounds. No synergistic effect was reported at 100 and 500 µg/mL for SIRT-3 (Figure 6).

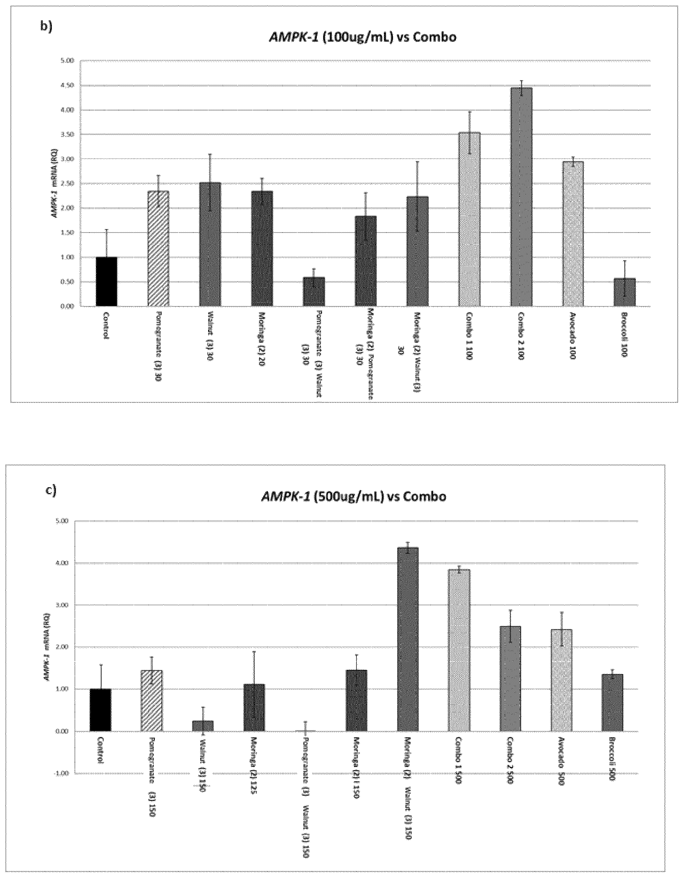

As regards to AMPK-1, an effect of the combinations was also reported at 100 µg/mL and 500 µg/mL, but synergistic only for combination 2 (Figure 9).

There was no synergistic effect on SIRT-1 (Figure 4) for both Broccoli and Avocado in the combinations 2:2:3, 1:1:1, 4:4:2, and 1:1:2.

As shown in Figure 7, a synergistic effect on SIRT-3 was found only for the Combo 1:1:2:2 for Broccoli.

As shown in Figure 10, a synergistic effect on AMPK1 was found for the Combo 1:1:2:2 with Broccoli and 2:2:3:3 with Avocado.

### Bibliographic References

1. Chomczynski P, Mackey K. Modification of the TRI reagent procedure for isolation of RNA from polysaccharide- and proteoglycan-rich sources. Biotechniques 1995;19:942-5.
2. Vigetti D, Viola M, Karousou E, Rizzi M, Moretto P, Genasetti A, et al. Hyaluronan-CD44-ERK1/2 regulate human aortic smooth muscle cell motility during aging. J Biol Chem 2008;283:4448-58.) was used for the calculation of the relative abundance in the expression of each gene.
3. Grabowska W, Sikora E, Bielak-Zmijewska A. Sirtuins, a promising target in slowing down the ageing process. Biogerontology. 2017;18(4):447-476.
4. Serravallo M, Jagdeo J, Glick SA, Siegel DM, Brody NI. Sirtuins in dermatology: applications for future research and therapeutics. Arch Dermatol Res. 2013 May;305(4):269-82.
5. Salminen A, Kaarniranta K. AMP-activated protein kinase (AMPK) controls the aging process via an integrated signaling network. Ageing Res Rev. 2012;11(2)-230-241.
6. Canto C, Gerhart-Hines Z, Feige JN, Lagouge M, Noriega L, Milne JC, Elliott PJ, Puigserver P, Auwerx J. AMPK regulates energy expenditure by modulating NAD+ metabolism and SIRT1 activity. Nature. 2009;458:1056-1060.
7. Zerr P, Palumbo-Zerr K, Huang J, Tomcik M, Sumova B, Distler O, Schett G, Distler JH. Sirt1 regulates canonical TGF-β signalling to control fibroblast activation and tissue fibrosis. Ann Rheum Dis. 2016 Jan;75(1):226-33.

## Claims

1. A composition comprising a basic vegetal nutritional mixture comprising Pomegranate (*Punica granatum L.*), Walnut (*Juglans nigra*) and Moringa *(Moringa oleifera Lam.*) combined with one further vegetal nutritional component selected from Avocado (*Persea americana Mill*) and Broccoli *(Brassica oleracea* var. italica), **characterized in that** the three nutritional components of the basic nutritional mixture are in ratios of 2.7-3.3: 2.7-3.3: 1.7-2.3, and the further vegetal nutritional component is in a ratio of 1.7-2.3.

2. The composition according to claim 1 comprising a vegetal nutritional mixture of Pomegranate, Walnut and Moringa combined with Broccoli.

3. The composition according to claim 2, wherein the Pomegranate:Walnut:Moringa:Broccoli vegetal components are in a ratio of 2.7-3.3 : 2.7-3.3 : 1.7-2.3 : 1.7-2.3, preferably of 3:3:2:2.

4. The composition according to claim 1 comprising a vegetal nutritional mixture of Pomegranate, Walnut and Moringa combined with Avocado.

5. The composition according to claim 4, wherein the Pomegranate:Walnut:Moringa:Avocado vegetal components are in a ratio of 2.7-3.3 : 2.7-3.3 : 1.7-2.3 : 1.7-2.3 , preferably of 3:3:2:2.

6. The composition according to any one of claims 1-5, wherein the vegetal nutritional components are in a grounded, blended or pulp/puree vegetable form.

7. The composition according to any one of claims 1-6 in a lyophilized and/or pasteurized form.

8. The composition according to any one of claims 1-7, wherein one or more of the vegetal components are in the form of an extract.

9. The composition according to claim 8 in a form for oral administration.

10. The composition according to claim 8 for topical application in the form of a cream, ointment, mask, compress or pulp/puree.

11. A nutraceutical or food supplement comprising a composition comprising Pomegranate (*Punica granatum L.*), Walnut (*Juglans nigra*) and Moringa (*Moringa oleifera Lam.) c*ombined with one further vegetal nutritional component selected from Avocado (*Persea americana Mill*) and Broccoli (*Brassica oleracea* var. italica), **characterized in that** the three nutritional components of the basic nutritional mixture are in ratios of 2.7-3.3: 2.7-3.3: 1.7-2.3, and the further vegetal nutritional component is in a ratio of 1.7-2.3.

12. The composition according to any one of claims 1-10 for use in prolonging the longevity of an individual and/or for keeping the biochemical parameters of the organism within the physiological parameters.

13. The composition for use according to claim 12, wherein said composition is in a form for oral administration.

14. The composition for use according to claim 12 further comprising a physiologically acceptable carrier and optionally a further biologically active ingredient selected from vitamins, minerals, micronutrients, plant extracts, and mixtures thereof.

## Patentansprüche

1. Zusammensetzung, umfassend eine pflanzliche Grundnährstoffmischung, die Granatapfel (*Punica granatum L.*), Walnuss (*Juglans nigra*) und Meerrettichbaum (*Moringa oleifera Lam.*) in Kombination mit einer weiteren pflanzlichen Nährstoffkomponente umfasst, die aus Avocado (*Persea americana Mill*) und Brokkoli (*Brassica oleracea* var. italica) ausgewählt ist, **dadurch gekennzeichnet, dass** die drei Nährstoffkomponenten der Grundnährstoffmischung in den Verhältnissen 2,7-3,3 : 2,7-3,3 : 1,7-2,3 vorliegen und die weitere pflanzliche Nährstoffkomponente in einem Verhältnis von 1,7-2,3 vorliegt.

2. Zusammensetzung nach Anspruch 1, umfassend eine pflanzliche Nährstoffmischung aus Granatapfel, Walnuss und Meerrettichbaum in Kombination mit Brokkoli.

3. Zusammensetzung nach Anspruch 2, wobei die pflanzlichen Komponenten Granatapfel/Walnuss/Meerrettichbaum/Brokkoli in einem Verhältnis von 2,7-3,3 : 2,7-3,3 : 1,7-2,3 : 1,7-2,3, vorzugsweise von 3 : 3 : 2 : 2, vorliegen.

4. Zusammensetzung nach Anspruch 1, umfassend eine pflanzliche Nährstoffmischung aus Granatapfel, Walnuss und Meerrettichbaum in Kombination mit Avocado.

5. Zusammensetzung nach Anspruch 4, wobei die pflanzlichen Komponenten Granatapfel/Walnuss/Meerrettichbaum/Avocado in einem Verhältnis von 2,7-3,3 : 2,7-3,3 : 1,7-2,3 : 1,7-2,3, vorzugsweise von 3 : 3 : 2 : 2, vorliegen.

6. Zusammensetzung nach einem der Ansprüche 1-5, wobei die pflanzlichen Nährstoffkomponenten in einer gemahlenen, pürierten oder pulpen-/ breiförmigen pflanzlichen Form vorliegen.

7. Zusammensetzung nach einem der Ansprüche 1-6 in einer lyophilisierten und/oder pasteurisierten Form.

8. Zusammensetzung nach einem der Ansprüche 1-7, wobei eine oder mehrere der pflanzlichen Komponenten in der Form eines Extrakts vorliegen.

9. Zusammensetzung nach Anspruch 8 in einer Form zur oralen Verabreichung.

10. Zusammensetzung nach Anspruch 8 zur topischen Anwendung in Form einer Creme, Salbe, Maske, Kompresse oder einer/s Pulpe/Breis.

11. Nutrazeutikum oder Nahrungsergänzungsmittel, umfassend eine Zusammensetzung, die Granatapfel *(Punica granatum L.),* Walnuss *(Juglans nigra)* und Meerrettichbaum *(Moringa oleifera Lam.)* in Kombination mit einer weiteren pflanzlichen Nährstoffkomponente umfasst, die aus Avocado (*Persea americana Mill*) und Brokkoli (*Brassica oleracea* var. italica) ausgewählt ist, **dadurch gekennzeichnet, dass** die drei Nährstoffkomponenten der Grundnährstoffmischung in den Verhältnissen 2,7-3,3 : 2,7-3,3 : 1,7-2,3 vorliegen und die weitere pflanzliche Nährstoffkomponente in einem Verhältnis von 1,7-2,3 vorliegt.

12. Zusammensetzung nach einem der Ansprüche 1-10 zur Verwendung zur Verlängerung der Lebensdauer einer Person und/oder zum Halten der biochemischen Parameter des Organismus innerhalb der physiologischen Parameter.

13. Zusammensetzung zur Verwendung nach Anspruch 12, wobei die Zusammensetzung in einer Form zur oralen Verabreichung vorliegt.

14. Zusammensetzung zur Verwendung nach Anspruch 12, die ferner einen physiologisch verträglichen Träger und gegebenenfalls einen weiteren biologisch aktiven Bestandteil umfasst, ausgewählt aus Vitaminen, Mineralstoffen, Mikronährstoffen, Pflanzenextrakten und Mischungen davon.

## Revendications

1. Composition comprenant un mélange nutritionnel végétal de base comprenant de la grenade (*Punica granatum L.*), de la noix (*Juglans nigra*) et du moringa (*Moringa oleifera Lam.*) combinés à un autre composant nutritionnel végétal choisi parmi l'avocat (*Persea americana Mill*) et le brocoli (*Brassica oleracea var. italica*), **caractérisée en ce que** les trois composants nutritionnels du mélange nutritionnel de base sont dans des rapports de 2,7-3,3: 2,7-3,3: 1,7-2,3, et l'autre composant nutritionnel végétal est dans un rapport de 1,7-2,3.

2. Composition selon la revendication 1, comprenant un mélange nutritionnel végétal de grenade, noix et moringa combiné avec du brocoli.

3. Composition selon la revendication 2, dans laquelle les composants végétaux Grenade:Noix:Moringa:Brocoli sont dans un rapport de 2,7-3,3 : 2,7-3,3 : 1,7-2,3 : 1,7-2,3, de préférence de 3:3:2:2.

4. Composition selon la revendication 1 comprenant un mélange nutritionnel végétal de grenade, noix et moringa combiné avec de l'avocat.

5. Composition selon la revendication 4, dans laquelle les composants végétaux Grenade:Noix:Moringa:Avocat sont dans un rapport de 2,7-3,3 : 2,7-3,3 : 1,7-2,3 : 1,7-2,3, de préférence de 3:3:2:2.

6. Composition selon l'une quelconque des revendications 1 à 5, dans laquelle les composants nutritionnels végétaux sont sous une forme végétale broyée, mélangée ou de pulpe/purée.

7. Composition selon l'une quelconque des revendications 1 à 6 sous une forme lyophilisée et/ou pasteurisée.

8. Composition selon l'une quelconque des revendications 1 à 7, dans laquelle un ou plusieurs des composants végétaux sont sous la forme d'un extrait.

9. Composition selon la revendication 8, qui est sous une forme pour l'administration par voie orale.

10. Composition selon la revendication 8 pour application topique sous forme de crème, pommade, masque, compresse ou pulpe/purée.

11. Produit nutraceutique ou complément alimentaire comprenant une composition comprenant de la grenade (*Punica granatum L.*)*,* de la noix (*Juglans nigra*) et du moringa (*Moringa oleifera Lam.*) combinés à un autre composant nutritionnel végétal choisi parmi l'avocat (*Persea americana Mill*) et le brocoli (*Brassica oleracea var. italica*), **caractérisé en ce que** les trois composants nutritionnels du mélange nutritionnel de base sont dans des rapports de 2,7-3,3: 2,7-3,3: 1,7-2,3, et l'autre composant nutritionnel végétal est dans un rapport de 1,7-2,3.

12. Composition selon l'une quelconque des revendications 1 à 10, destinée à être utilisée pour prolonger la longévité d'un individu et/ou pour maintenir les paramètres biochimiques de l'organisme dans les paramètres physiologiques.

13. Composition à utiliser selon la revendication 12, dans laquelle ladite composition est sous une forme permettant l'administration par voie orale.

14. Composition à utiliser selon la revendication 12, comprenant en outre un support physiologiquement acceptable et éventuellement un autre ingrédient biologiquement actif choisi parmi les vitamines, les minéraux, les micronutriments, les extraits de plantes et leurs mélanges.
